Europäisches Patentamt

European Patent Office

Office européen des brevets

(11) Publication number: **0 194 060 B1**

# EUROPEAN PATENT SPECIFICATION

(45) Date of publication of patent specification: **28.08.91**  (51) Int. Cl.⁵: **A23L 1/308, A61K 35/78**

(21) Application number: **86301020.3**

(22) Date of filing: **14.02.86**

(54) Desmutagenic substances and their production.

(30) Priority: **16.02.85 JP 28918/85**

(43) Date of publication of application:
**10.09.86 Bulletin 86/37**

(45) Publication of the grant of the patent:
**28.08.91 Bulletin 91/35**

(84) Designated Contracting States:
**DE FR GB**

(56) References cited:
**EP-A- 0 117 044**
**EP-A- 0 166 824**
**US-A- 4 181 747**
**US-A- 4 307 121**

**PATENTS ABSTRACTS OF JAPAN, vol. 6, no.
106 (C-108)[984], 16th June 1982; & JP-A-57
36947 (NIPPON SHIYOKUHIN KAKOU K.K.)
27-02-1982**

**PATENTS ABSTRACTS OF JAPAN, vol. 9, no.
45 (C-268)[1768], 26th February 1985; & JP-
A-59 187 745 (NIHON SHIYOKUHIN KAKOU
K.K.) 24-10-1984**

(73) Proprietor: **Nihon Shokuhin Kako Co., Ltd.
No. 4-1, 3-chome, Marunouchi
Chiyoda-ku Tokyo(JP)**

(72) Inventor: **Kada, Tsuneo
27 Toyotamakita 5-Chome
Nerimaku Tokyo(JP)**
Inventor: **Takeuchi, Masayasu
2954 Imaizumi
Fujishi Shizuokaken(JP)**
Inventor: **Kawamura, Saburo
29-9 Higashitoyoda 1-Chome
Hinoshi Tokyo(JP)**
Inventor: **Miwa, Taizo
28-3 Sagamidai 2-Chome
Sagamiharashi Kanagawaken(JP)**

(74) Representative: **Skailes, Humphrey John et al
Frank B. Dehn & Co. Imperial House 15-19
Kingsway
London WC2B 6UZ(GB)**

## Description

BACKGROUND OF THE INVENTION

1. Field of the Invention

The present invention relates to desmutagenic substances and their production. The desmutagenic substances according to the present invention can effectively inactivate mutagens, namely, substances capable of inducing mutation such as nitroallene or pyrolysate mutagens of food.

2. Description of the Prior Art

Recently, as a result of the finding that mutagens have a strong relation to carcinogenesis, studies of mutagens have been zealously made by researchers in food or related industries. The mutagens, as is well recognized in the art, have a function of inducing mutation by acting on DNA or deoxyribonucleic acid.

The mutagens or the substances capable of prcviding such mutagens occur everywhere in our daily life. One of them is an environmental pollutant such as nitroallene or the like, typically dinitropyrene, for example. Another one is a pyrolysate mutagen of food such as amino acid contained therein. Other mutagens include, for example, browning substance produced during cooking of food, tar of tobaccos and the like. These substances are generally recognized to be very strong mutagens or mutagen-providing substance.

Particularly, nitroallene, which has been recently found in exhaust gas of diesel engines, along with benzopyrene, necessitates countermeasures to inactivate their mutagenicity. This is because, among numerous environmental pollutants, they have the strongest carcinogenicity as a result of their high mutagenicity. It is reported in the literature that nitroallene is a remarkably stable substance and, in animal experiments, it can cause cancer in an amount of about 80 ng/kg.

Hitherto, there have been proposed several methods which are considered to be effective for inactivating the mutagens described above. For instance;

(1) Use of Vitamin C

This will inactivate the reaction product of cysteine or sorbic acid with a nitrous acid or an insecticide such as captan, i.e., N-trichloromethylthiotetrahydrophthalimide.

(2) Use of Peroxidase

Peroxidases which are naturally occurring in vegetables will inactivate pyrolysates of tryptophan (Trp-P-1; a kind of pyrolysate mutagen).

(3) Inhibition of a Formation of Carcinogen Intermediates

In the case where a metabolic intermediate of a chemical carcinogen is toxic, the mutagens will be inactivated if the formation of such intermediate is inhibited or prevented.

(4) Utilization of Scavenging Action

A fibrous material of vegetables can effectively act as a so-called "scavenger". It can adsorb the combustion product of tryptophan, thereby inactivating the mutagens.

(5) Use of Specially Prepared Cotton Products

A specially prepared cotton product having fixed therein copper phthalocyanine trisulfonic acid is used to detect any mutagen in an environment. This cotton product can adsorb benzo ($\alpha$) pyrene, thereby inactivating the mutagens.

However, these prior art methods are not satisfactory to inactivate the mutagens, because they are frequently accompanied with drawbacks which will be described below. For example, when vitamin C or peroxidases, both occurring in the vegetables, are used for an inactivation purpose, heat applied during cooking will decompose vitamin C and cause inactivation of peroxidases. This means that an desmutagenic activity of the vitamin C and peroxidases is lost during cooking in many cases.

Further, uptake of fibrous vegetables such as burdock, cabbage and carrot as a scavenger is not practical. In fact, it is difficult to eat a large volume of vegetables at every meal or, alternatively, to eat a small amount of vegetables at frequent intervals throughout the day. In any case, insufficient uptake of the fibrous vegetables will not result in satisfactory inactivation of mutagens such as pyrolysates of tryptophan.

Furthermore, use of copper phthalocyanine trisulfonic acid or similar compounds proposed in the method (5) should be avoided, since they are toxic to the human body. Of course, they are not ingestable. Finally, it is noted that inactivation of nitroallene with a food component or element has not been taught or suggested in the art as yet.

Under these circumstances, it is now desirable to solve the above-discussed problems of the prior art methods and to provide a novel desmutagenic substance which is a naturally occurring substance having no toxicity to the human body, a small amount of which substance is sufficient to inactivate the mutagens, and an inactivation of which substance is not hindered or adversely affected with the application of heat.

## SUMMARY OF THE INVENTION

According to the present invention, there is provided a novel desmutagenic substance consisting of a fibrous constituent of food produced from corn fibres and contains as its principal component hardly digestible substances separated from the corn fibres. The term "hardly digestible" used herein is intended to indicate that the substances have no or little digestability. The substance has a NDF or Neutral Detergent Fibre value of 50% or more and a particle size of not larger than 16 meshes.

According to the present invention, there is also provided a new process for the production of desmutagenic substances which comprises subjecting corn fibres as a starting material to an enzyme treatment, chemical treatment or physical treatment or a combination thereof to remove starch, protein or other digestibles therefrom, thereby producing a fibrous food constituent having a Neutral Detergent Fibre (NDF) value of 50% or more and finely dividing the fibrous constituent and recovering material having a particle size of not more than 16 mesh which contains as its principal component hardly digestible substances containing hemicellulose and cellulose.

The enzyme treatment of the corn fibres in the production process according to the present invention is preferably carried out using one or more amylolytic, proteolytic, lipolytic or cellulolytic enzymes or a complex enzyme.

The chemical treatment of the corn fibres in the present process is preferably carried out using an aqueous solution of alkali or acid or, alternatively, with the addition of a surfactant or surface activating agent.

The physical treatment of the corn fibres is preferably performed using a grinding machine and a sifting machine.

As will be apparent from the below-described preferred embodiments of the present invention and the appended working example, the desmutagenic substances of the present invention, even though they are used in very small quantity, can show a remarkably strong desmutagenic action against a variety of mutagens such as nitroallene, pyrolysate mutagens or the like. Surprisingly, the desmutagenic action of the desmutagenic substances is not hindered even if they are exposed to heat. In addition, these desmutagenic substances are non-toxic to the human body, because they are a naturally occurring substance. These substances can also exhibit a preventive or prophylactic effects against cancer, which effect is considered to result from their low digestability and short transition time in the human body. In this connection, it is also considered that an injurious action of the mutagens on the body is completely blocked by the above characteristics of the present desmutagenic substances.

## DESCRIPTION OF THE PREFERRED EMBODIMENTS

In the practice of the present invention, corn fibres are used as a starting material. The term "corn fibres" has a meaning generally applied thereto, and indicates the hull or shell portion of corn (Zea mays L.). The corn fibres can be conveniently separated from the corns using a dry milling process or a wet milling process; both are conventional. The dry milling process comprises crushing the corns under dry conditions, while the wet milling comprises dipping the corns in a solution of sulfurous acid, milling the wetted corns and sifting the milled corns to separate hulls therefrom.

The corn fibres are treated to remove starch, protein or other unnecessary components therefrom. As the treating process for this purpose, an enzyme treatment, chemical treatment or physical treatment may be used separately or in combination. As a result of this treatment, a fibrous constituent of food or fibrous food which contains as its principal component hardly digestible or indigestible substances such as cellulose, hemicellulose or similar substances is produced. The resulting fibrous constituent of food constitutes a desmutagenic substance of the present invention.

As described above, one of the useful treatment processes is an enzyme treatment. The enzyme treatment comprises adding to the corn fibres one or more amylolytic enzymes, proteolytic enzymes, lipolytic enzymes or cellulolytic enzymes at a pH value of about 3 to 9 and at a temperature of about 30 to 100°C. Typical examples of useful enzymes are described hereinbelow, but the present invention is not limited to these enzymes: the amylolytic enzymes include α-amylase, glucoamylase or the like, the proteolytic enzymes include protease or the like, the lipolytic enzymes include lipase or the like, and the cellulolytic enzymes include cellulose or the like. If desired, a combined or complex enzyme which can

EP 0 194 060 B1

concurrently contain an amylolytic enzyme, a proteolytic enzyme and a lipolytic enzyme, for example, pancreatin, can be used in the present treatment process.

As an alternative, the corn fibres can be chemically treated with an aqueous solution of alkali or acid, or with the addition of a surface activating agent. Typical examples of alkali usable in this treatment include sodium hydroxide, potassium hydroxide, calcium hydroxide, sodium carbonate or the like. Useful acids include both of mineral acids such as hydrochloric acid, sulfuric acid, phosphoric acid or the like, and organic acids such as acetic acid, lactic acid or the like, for example. Further, useful surface activating agents include, for example, monoglyceride, sugar ester, sodium lauryl sulfate or the like.

Moreover, the corn fibres can be physically treated using conventional methods and machines. For instance, they can be pulverized with a grinding machine such as a homogenizer or a hammer mill, and then sifted using a sieve with appropriate openings to remove easily crushed starch, protein or similar materials.

We found that an desmutagenic activity of the desmutagenic substances according to the present invention is more increased with increase of a surface area of the substances. It is, therefore, necessary that the present desmutagenic substances have a particle size of not larger than 16 mesh. A particle size exceeding 16 mesh should be avoided, because the substances having such large particle size exhibit insufficient desmutagenic activity as a result of decrease of the surface area, and, in addition, they are not smoothly passed through the esophagus after eating. The latter means an inferior and unacceptable sense or feeling in eating.

In addition to the particle size of the desmutagenic substances, a NDF or Neutral Detergent Fibre value of the fibrous constituent of food in said substances is also important, and should be 50% or more, in terms of anhydride. This is because, in order to inhibit the function of the mutagens in the human body, namely, in order to discharge the mutagens out of the body, it is preferred to control the amount of digestible substances such as starch and protein to a level as low as possible.

From our studies for the present desmutagenic substances up to date, it is believed that the inactivation of the mutagens according to the present invention largely depends on the existence in particular of the hardly digestible substances in the corn fibres, for example, hemicellulose, lignin, inositol, phosphor compounds, metals or others in addition to cellulose, and therefore satisfactory inactivation of the mutagens can be attained even with the use of a very small amount of the present substances, if these substances contain a large amount of the hardly digestible substances and they are finely divided to provide a large surface area.

The thus resulting desmutagenic substances of the present invention are naturally occurring and edible by themselves and, as previously described, a very small amount is sufficient to attain the intended purpose. Therefore, these substances can be widely used alone or, alternatively, as a constitutional element in the production of the generally used processed foods, health foods, medicines or similar products.

In the practice of the present invention, desmutagenic activity to the mutagens provided by the desmutagenic substances can be evaluated using a conventional mutation test, for example, Ames's method described on pages 56 to 67 of "An Experimental Method for Environmental Mutagens" published by KODAN-SHA LTD., Tokyo, in 1980. The Ames's method will be briefly described hereinafter.

The mutation test based on the Ames's method has another name: Salmonella test. This test comprises the steps of disseminating a strain of Salmonella having a histidine requirement in an agar medium containing only a notably small amount of histidine and then counting the numbers of the growing colonies to determine the mutation rate or level of mutation.

More particularly, the above mutation test can be performed as follows. Assuming that the mutagen in question is dinitropyrene, 5 to 200 mg of the desmutagenic substance of the present invention is added to 1 ml of an aqueous solution of dinitropyrene, whose concentration is, for example, 0.1 $\mu$g/ml, and then left to stand at a room temperature for 1 to 3 hours. Thereafter, the mixture is centrifuged to separate only a supernatant. A predetermined amount (for example, 20 $\mu$l) of the supernatant is added to 3 ml of soft agar. The resulting soft agar medium is then used in the Ames's method-based mutation test, after a strain having histidine requirement, for example, Salmonella TA-98 strains, is added to the agar medium. Percentage (%) inactivation of the mutagens can be calculated from the following equation:

$$\% \ \text{inactivation} = \frac{(A - B)}{A} \times 100$$

in which

4

A is the colony count for the mutants formed when an aqueous solution of dinitropyrene which was not previously treated with the present desmutagenic substance was added to the agar medium, and

B is the colony count for the mutants formed when an aqueous solution of dinitropyrene previously treated with the present desmutagenic substance was added to the medium. For this experiment, in order to provide the same environmental conditions as in vivo of the animals, a liquid preparation extracted from the liver of rat or rabbit, for example, so-called "S-9 Mix", may be added to the soft agar medium.

The following examples are included to further illustrate the present invention.

Example 1

Corn was crushed with a hammer mill to separate hull portions. The hulls were washed in water, gathered using a wire gauze (12 mesh), hydro-extracted and dried in air. After drying, the hulls were milled to a particle size of not more than 32 mesh. A sample with a NDF value of 60% was obtained.

100 mg of the sample was added to 1 ml of an aqueous solution of dinitropyrene (0.1 $\mu$g/ml), and the mixture was then left to stand for 3 hours. Thereafter, the mixture was centrifuged to separate its supernatant liquid. 20 $\mu$l of the separated supernatant was added to a soft agar which was then used in the Ames's method-based mutation test. The test was performed using Salmonella TA-98 strains with a histidine requirement. The percentage inactivation of 89.1% was resulted.

Example 2

Corn was wet-milled in accordance with the conventional manner, and the resulting suspension of corn fibres was treated using a high speed homogenizer. The corn fibres were recovered through a sieve (32 mesh), washed with fresh warm water, and then dried in an air draft at 60°C. The corn fibres were further finely divided using a pulverizer, until the corn fibres having a particle size of not more than 200 mesh constitute 90% or more of the total amount. A sample with a NDF value of 88% was obtained.

30 mg or 100 mg of the thus obtained sample were each added to 1 ml of an aqueous solution of dinitropyrene (0.1 $\mu$g/ml), and the mixture was left to stand at a room temperature for 2 hours. Thereafter, as in the above Example 1, the supernatant liquid was separated, and the mutation test was performed. As a control, the above procedure was repeated without addition of dinitropyrene. The results are shown in the following Table 1.

## Table 1

| Sample | Number of mutants* | % inactivation |
|---|---|---|
| dinitropyrene (0.1 μg/ml, control) | 2231 | - |
| dinitropyrene (0.1 μg/ml) plus sample (30 mg) | 322 | 85.6 |
| dinitropyrene (0.1 μg/ml) plus sample (100 mg) | 8 | 99.6 |

* Number of mutants was evaluated by subtracting the colony count for the case containing no mutagen from the experimental results.

The above results indicate that the sample substance of the present invention, even though it is used in a small amount such as only 30 mg, can effectively inactive the mutagenicity of dinitropyrene.

Example 3

Corn was first dry-milled. The resulting corn hulls were then roughly milled and passed through a sieve (20 mesh) to recover corn fibres. The corn fibres were then finely divided using a pulverizer to a grain size of not more than 100 mesh.

5 mg, 10 mg and 20 mg portions of the resulting sample were added to 1 ml of 0.1 $\mu$g/ml of Glu-p-1 which is a kind of pyrolysate mutagen. After being left at a room temperature for 2 hours, the sample was centrifuged and the separated supernatant was filtered through a milipore filter having a pore size of 0.45 $\mu$m.

The Ames's method-based mutation test was performed using 0.1 ml of the filtrate. In this example, 0.1 ml of a liquid preparation S-9 Mix extracted from the liver of rabbit was added to the soft agar medium. The results are shown in the following Table 2.

## Table 2

| Sample | Number of mutants* | % inactivation |
|---|---|---|
| Glu-p-1 (0.1 µg/ml, control) | 661 | – |
| Glu-p-1 (0.1 µg/ml) plus sample (5 mg) | 243 | 63.2 |
| Glu-p-1 (0.1 µg/ml) plus sample (10 mg) | 108 | 83.7 |
| Glu-p-1 (0.1 µg/ml) plus sample (20 mg) | 120 | 81.8 |

\* see Table 1

The above results indicate that the sample substance produced from the corn fibres according to the present invention has excellent desmutagenic activity against the pyrolysate mutagens. This means that, when eating grilled meat which contains pyrolysate mutagens on its surface, we can effectively prevent formation of carcinomas due to the pyrolysate mutagens, if we concurrently take a very small amount of corn fibres.

Example 4

Corns was first subjected to a wett milling process. The resulting milled corn hulls were slurried, heated to 90° C, and mixed with "KleistaseT-5" (trade name; amylolytic enzyme commercially available from Daiwa Kasei Kogyo Kabushiki Kaisha). After reaction for 2 hours, the reaction product was then mixed with "BIOPLASE SP" (trade name; proteolytic enzyme commercially available from Nagase & Co., Ltd.), and reacted for further 5 hours. Starch, protein and other digestible substances were removed. The residue was washed with water, recovered, and dried in a draft of air. After the drying was completed, the solid substance was further finely divided, and passed through a sieve (60 mesh). The resultant powdered sample had a NDF value of 87%.

5 mg, 10 mg and 20 mg portions of the powdered sample were added to 1 ml of 1 $\mu$g/ml of Trp-p-1 (a kind of the pyrolysate mutagen). The mixture was left to stand at a room temperature for 2.5 hours. As in Example 1 above, a supernatant was separated, and 0.1 ml of the supernatant was subjected to the Ames's method based mutation test. In this example, as in the above Example 3, 0.1 ml of a liquid preparation of S-9 Mix was added to the soft agar medium. The results are summarized in the Table 3.

## Table 3

| Sample | Number of mutants* | % inactivation |
|---|---|---|
| Trp-p-1 (1 µg/ml, control) | 809 | -- |
| Trp-p-1 (1 µg/ml) plus sample (5 mg) | 20 | 97.5 |
| Trp-p-1 (1 µg/ml) plus sample (10 mg) | 15 | 98.1 |
| Trp-p-1 (1 µg/ml) plus sample (20 mg) | 34 | 95.8 |

\* see Table 1

The above results indicate that the sample substance according to the present invention has a high desmutagenic activity against the pyrolysate mutagen Trp-p-1 and that the satisfactory desmutagenic activity can be attained even with the use of a very small amount of the substance of the present invention.

## Claims

1. A desmutagenic substance which is a fibrous food constituent which has a NDF (Neutral Detergent Fibre) value of 50% or more and a grain size of not larger than 16 mesh (Japanese standard), and contains as its principal component hardly digestible substances (containing hemicellulose and cellulose) separated from corn fibres.

2. A process for the production of a desmutagenic substance according to claim 1 which comprises subjecting corn fibres as a starting material to an enzyme treatment, chemical treatment or physical treatment or a combination thereof to remove starch, protein or other digestible material therefrom, thereby producing a fibrous food constituent having a Neutral Detergent Fibre (NDF) value of 50% or more and finely dividing the fibrous constituent and recovering material having a particle size of not larger than 16 mesh (Japanese standard) which contains as its principal component hardly digestible substances containing hemicellulose and cellulose.

3. A process according to claim 2 in which the enzyme treatment of corn fibres is carried out using one or more amylolytic, proteolytic, lipolytic or cellulolytic enzymes or a complex enzyme which can concurrently contain amylolytic enzyme, a proteolytic enzyme and a lipolytic enzyme.

4. A process according to claim 2 in which the chemical treatment of corn fibres is carried out using an aqueous solution of alkali or acid.

5. A process according to claim 2 in which the chemical treatment of corn fibres is carried out with the addition of a surface activating agent.

6. A process according to claim 2 in which the physical treatment of corn fibres is carried out using a grinding machine and a sifting machine.

7. A food product or pharmaceutical composition which contains as an additive a desmutagenic substance according to claim 1 or when produced by a method according to any of claims 2 to 6.

8. The use of a desmutagenic substance according to claim 1 or when produced by a method according to any of claims 2 to 6 in a food product or pharmaceutical composition according to claim 7.

EP 0 194 060 B1

**Revendications**

1. Substance anti-mutagène qui est un constituant alimentaire fibreux ayant un indice NDF (Fibre de Détergent Neutre) de 50 % ou plus et une taille particulaire non supérieure à 16 mesh (norme japonaise), et contenant en tant que composant principal des substances difficilement digestibles (contenant de l'hémicellulose et de la cellulose) séparées à partir de fibres de maïs.

2. Procédé pour la production d'une substance anti-mutagène selon la revendication 1, dans lequel on soumet des fibres de maïs en tant que produit de départ à un traitement enzymatique, un traitement chimique ou un traitement physique, ou une combinaison de ceux-ci de manière à éliminer l'amidon, les protéines ou autres matières digestibles de manière à produire un constituant alimentaire fibreux ayant un indice de Fibre de Détergent Neutre (NDF) de 50 % ou plus et à diviser finement le constituant fibreux et à récupérer une matière ayant une dimension particulaire non supérieure à 16 mesh (norme japonaise) qui contient en tant que composant principal des substances difficilement digestibles contenant de l'hémicellulose et de la cellulose.

3. Procédé selon la revendication 2, dans lequel le traitement enzymatique des fibres de maïs est réalisé en utilisant une ou plusieurs enzymes amylolytique, protéolytique, lipolytique ou cellulolytique, ou un complexe enzymatique pouvant contenir simultanément une enzyme amylolytique, une enzyme protéolytique et une enzyme lipolytique.

4. Procédé selon la revendication 2, dans lequel le traitement chimique des fibres de maïs est réalisé en utilisant une solution aqueuse alcaline ou acide.

5. Procédé selon la revendication 2, dans lequel le traitement chimique des fibres de maïs est effectué avec l'addition d'un agent tensio-actif.

6. Procédé selon la revendication 2, dans lequel le traitement physique des fibres de maïs est effectué en utilisant une broyeuse et un tamis.

7. Produit alimentaire ou composition pharmaceutique contenant, en tant qu'additif, une substance anti-mutagène selon la revendication 1 ou produite par un procédé selon l'une quelconque des revendications 2 à 6.

8. Utilisation d'une substance anti-mutagène selon la revendication 1 ou produite par un procédé selon l'une quelconque des revendications 2 à 6, pour la réalisation d'un produit alimentaire ou d'une composition pharmaceutique selon la revendication 7.

**Patentansprüche**

1. Desmutagene Substanz, welche ein faserförmiger Nahrungsmittelbestandteil ist, der einen NDF (neutralen Detergenzfaser)-Wert von 50% oder mehr und eine Korngröße von nicht höher als 16 mesh (japanischer Standard) besitzt und als wesentliche Komponente aus Korn- bzw. Getreidefasern abgetrennte schwerverdauliche Substanzen (enthaltend Hemicellulose und Cellulose) enthält.

2. Verfahren zur Herstellung einer desmutagenen Substanz gemäß Anspruch 1, bei dem man Korn-bzw. Getreidefasern als Ausgangsmaterial einer Enzymbehandlung, chemischen Behandlung oder physikalischen Behandlung oder einer Kombination hiervon zur Entfernung von Stärke, Protein oder anderem verdaubaren Material hieraus unterzieht, wobei man einen faserförmigen Nahrungsmittelbestandteil mit einem neutralen Detergenzfaser (NDF)-Wert von 50% oder mehr bildet, und den faserförmigen Bestandteil fein zerteilt und ein Material mit einer Teilchengröße von nicht höher als 16 mesh (japanischer Standard) gewinnt, das als Hauptkomponente Hemicellulose und Cellulose enthaltende schwer verdaubare Substanzen enhält.

3. Verfahren gemäß Anspruch 2, bei dem die Enzymbehandlung der Korn- bzw. Getreidefasern unter Verwendung eines oder mehrerer amylolytischer, proteolytischer, lipolytischer oder cellulolytischer Enzyme oder eines komplexen Enzyms, welches gleichzeitig ein amylolytisches Enzym, ein proteolytisches Enzym und ein lipolytisches Enzym enthalten kann, durchgeführt wird.

8

4. Verfahren gemäß Anspruch 2, bei dem die chemische Behandlung der Korn- bzw. Getreidefasern unter Verwendung einer wäßrigen Lösung von Alkali oder Säure durchgeführt wird.

5. Verfahren gemäß Anspruch 2, bei dem die chemische Behandlung der Korn- bzw. Getreidefasern unter Zusatz eines oberflächenaktiven Mittels durchgeführt wird.

6. Verfahren gemäß Anspruch 2, bei dem die physikalische Behandlung der Korn- bzw. Getreidefasern unter Verwendung einer Schleifmaschine und einer Siebmaschine durchgeführt wird.

7. Nahrungsmittelprodukt oder pharmazeutische Zusammensetzung, die als Additiv eine desmutagene Substanz gemäß Anspruch 1 oder hergestellt nach einem Verfahren gemäß einem der Ansprüche 2 bis 6 enthält.

8. Verwendung einer desmutagenen Substanz gemäß Anspruch 1 oder hergestellt nach einem Verfahren gemäß einem der Ansprüche 2 bis 6 in einem Nahrungmittelprodukt oder einer pharmazeutischen Zusammensetzung gemäß Anspruch 7.